# EUROPEAN PATENT APPLICATION

(11) **EP 2 617 422 A1**
(43) Date of publication of application: **24.07.2013**
(21) Application number: 12151996.1
(22) Date of filing: 20.01.2012
(51) Int. Cl.: A61K 31/519, A61P 35/00

(54) **Anti-tumor activity of reduced folates like methylene-tetrahydrofolate, tetrahydrofolate or methyl-tetrahydrofolate**

(71) Applicant: Isofol Medical AB, 413 46 Gothenburg (SE)
(72) Inventor: Gustavsson, Bengt, 426 79 Västra Frölunda (SE); Carlsson, Björn, 426 79 Västra Frölunda (SE)
(74) Representative: Bergh, Johanna

(57) **Abstract**

The present invention relates to the use of reduced folates such as methylene-tetrahydrofolate, tetrahydrofolate or methyl-tetrahydrofolate for use in the treatment of cancer, based on their intrinsic antitumor activity. The cancers that may be treated include breast cancer, gastric cancer, gastrointestinal cancer, gall bladder cancer, bile duct cancer, colon cancer, rectal cancer, liver cancer, pancreatic cancer, head and neck cancer, esophageal cancer, mesotheolioma cancer, lung cancer including non-small-cell lung cancer, ovarian cancer, endometrial cancer, cervicial cancer, peripheral T-cell lymphoma (PTCL), melanoma, brain tumors, adenocarcinoma, esophageal cancer, and osteosarcoma.

## Description

### Field of the invention

The present invention relates to reduced folates for the treatment of cancer.

### Background of the invention

Cancer is one of the world's largest health concerns. It is one of the major leading causes of deaths worldwide and together with cardiovascular diseases, diabetes, chronic respiratory diseases it causes over 60% of all deaths globally. Nearly 12.7 million new cancer cases and 7.6 million cancer deaths occurred in 2008 worldwide.

Colorectal cancer is the third most common cancer in men and the second in women worldwide. Almost 60% of the cases occur in developed regions. Incidence rates vary worldwide, but are overall substantially higher in men than in women. Over 600 000 people die each year from the disease, accounting for 8% of all cancer-related deaths. In the US alone, over 150 000 new cases are diagnosed each year. Colon cancer is defined as cancer that forms in the tissues of the colon (the longest part of the large intestine). Most colon cancers are adenocarcinomas (cancers that begin in cells that make and release mucus and other fluids).

Cancer of the colon is a highly treatable and often curable disease when localized to the bowel. Surgery is the primary form of treatment and results in cure in approximately 50% of the patients. Recurrence following surgery is a major problem and is often the ultimate cause of death. Nearly half of these colorectal cancer cases are metastatic or develop into a metastasized disease. In these cases, chemotherapy is the sole treatment option and the prognosis for the patient is often rather poor. Similarly, treatment regimens for other forms of cancer do not lead to full recovery of all patients and many cancers recur and/or develop into metastatic forms.

There is therefore a great need for new and improved drug-based therapies to combat not only colorectal cancer, but also a number of other cancer indications such as, for example, breast cancer, gastric cancer, gastrointestinal cancer, gall bladder cancer, bile duct cancer, liver cancer, pancreatic cancer, head and neck cancer, esophageal cancer, mesotheolioma cancer, lung cancer including non-small-cell lung cancer, ovarian cancer, endometrial cancer, cervicial cancer, peripheral T-cell lymphoma (PTCL), melanoma, brain tumors, adenocarcinoma, esophageal cancer, and osteosarcoma.

Folates are endogenous substances that are essential for cell division and cell growth. Intracellular reduced folates exist as a pool of at least six interconvertable forms. Folates are for instance involved in nucleotide metabolism, where they serve as substrates and/or coenzymes of various enzymes, such as thymidylate synthase (TS) and dihydrofolate reductase (DHFR). The folate methylene-tetrahydrofolate (methylene-THF), for example, acts as a one-carbon donor in the conversion between dUMP and dUTP, performed by TS. Thereby it contributes to the synthesis of thymidine and thus to the synthesis of DNA and to cell growth.

The role of folates in carcinogenesis is complex. Experimental data suggest that the timing of folate supplementation during carcinogenesis is of importance (Ulrich, Cancer Epidemiol Biomarkers Prev (2006), vol. 15, pages 189 -93; Kim, Gut (2006), vol. 55, pages 1387-1389). Although increases in folate levels before the existence of preneoplastic lesions (such as aberrant crypt foci or polyps in the colon) can prevent tumor development, folate supplementation is believed to enhance cancer progression once preneoplastic lesions are present. Thus, folates are believed to inhibit cancer development when used preventively, but believed to enhance cancer progression once cancer has started to develop. Since folates are known to be involved in synthesis of nucleotides and in cell growth, it has been expected that they have such cancer promoting effect. Furthermore, cancer cells frequently up-regulate folate receptors to meet their elevated need for nucleotides to support DNA synthesis and growth, hence an increased risk of tumor growth promotion with folate administration is expected (Ulrich, Am. J. Clin. Nutr. (2007), vol. 86, pages 271-273).

Antifolates, such as methotrexate and pemetrexed, have thus been used as chemotherapeutic agents for the treatment of cancer, by being able to inhibit one or more of the enzymes involved in the folate and nucleotide metabolism, e.g. TS and/or DHFR. Fluoropyrimidines, such as 5-fluorouracil (5-FU), has similarly been used as chemotherapeutic agents, by being able to inhibit TS. Antifolates as well as fluoropyrimidines are however cytotoxic and can be associated with severe side effects for many patients.

Folic acid and folates, such as in the form of leucovorin (also known as folinic acid), levoleucuvorin and methylene-THF, have been co-administered with chemotherapeutic agents to cancer patients. Folates have for example been used as rescue agents to methotrexate, in order to reduce the toxic side effects of the methotrexate treatment. Folic acid, leucovorin and methylene-THF have also been used in combination with 5-FU, in order to enhance the anti-tumoral effect of 5-FU.

US 5 376 658 (Spears et al) discloses the use of tetrahydrofolate (FH₄), and its equilibrium product in solution methylene-THF (CH₂FH₄), as a modulator of 5-FU in cancer chemotherapy. Also disclosed is a method of using FH₄ or CH₂FH₄ in order to reduce the toxicity of an antifolate drug which has been administered to a patient.

US 2007/0099866 A1 and WO 2007/064968 disclose the use of methylene-THF to enhance the chemotherapeutic effect of 5-FU.

Borsi et al (Pediatric Hematology and Oncology 1990, vol. 7, pages 347-363) disclose the use of leucovorin and methylene-THF as rescue agents in connection with methotrexate treatment, to alleviate the sometimes very toxic side effects of this antifolate.

EP 1 699 462 B1 discloses the use of methyl-THF in co-administration with multi-targeting antifolates, such as pemetrexed, in order to reduce the side effects of the multi-tageting antifolate while maintaining or improving the anti-cancer efficiency.

Thus, folates, such as leucovorin and methylene-THF, have been used together with different chemotherapeutic agents, in order to modulate their cytotherapeutic effect or to alleviate their toxic side effects. Folates, such as methylene-THF, have in themselves, however, since long been known to propagate cell growth and has thus in themselves been expected to have a cancer promoting effect.

### Summary of the invention

The basis for this invention is the surprising finding that methylene tetrahydrofolate (methylene-THF) has been discovered to have a marked intrinsic tumor inhibiting activity when used as single agent. As shown in this disclosure, the present inventors have clearly demonstrated this anti-tumor efficacy in a nude mice xenograft model. The finding is especially surprising considering the inherent cell propagating characteristics of folates. The present invention demonstrates a novel use of methylene-THF as an anti-cancer agent when used in isolation of other cancer drugs.

There is a great difference in metabolism between tumor tissue, surrounding mucosa and healthy cells. The present invention further demonstrates that use of methylene-tetrahydrofolate may increase the therapeutic index by providing a marked tumor-inhibition effect while simultaneously protecting healthy cells.

In one aspect the present invention provides methylene-tetrahydrofolate, tetrahydrofolate or methyl-tetrahydrofolate for use in the treatment of cancer. The methylene-tetrahydrofolate, tetrahydrofolate or methyl-tetrahydrofolate may be administered as a single agent treatment or in isolation from other anti-cancer drugs. It may be used to inhibit tumor growth or reduce the volume of a tumor. The tumor may be solid or a non-solid tumor, preferably a solid tumor.

In one embodiment the methylene-tetrahydrofolate, tetrahydrofolate or methyl-tetrahydrofolate is for administration no later than 24 hours before the administration of any other anti-cancer agent. In other embodiments it is for administration no later than 36 hours or 48 hours before the administration of any other anti-cancer agent.

In another embodiment the methylene-tetrahydrofolate, tetrahydrofolate or methyl-tetrahydrofolate is for administration at the earliest 6 hours after the administration of any other anti-cancer agent, or at the earliest 24 hours, 48 hours, 4 days or 12 days after the administration of any other anti-cancer agent.

In still another embodiment methylene-tetrahydrofolate, tetrahydrofolate or methyl-tetrahydrofolate is for administration in isolation from other anti-cancer drugs during a time period of at least 3 months.

The methylene-tetrahydrofolate, tetrahydrofolate or methyltetrahydrofolate may be for administration in an amount of at least 10 mg/m², preferably at least 20 mg/m², more preferably at least 50 mg/m², most preferably at least 100 mg/m², 200 mg/m² or 500 mg/m², and at maximum 5 g/m². It may be for administration once or twice daily, every second or third day, once to twice a week, or once every second or third week.

The methylene-tetrahydrofolate, tetrahydrofolate or methyltetrahydrofolate may for instance be for administration intravenously, orally, intraperitoneally, subcutaneously, intramuscularly, intra-arterially or intranasally.

The cancer to be treated by the methylene-tetrahydrofolate, tetrahydrofolate or methyl-tetrahydrofolate of the present invention may be selected from breast cancer, gastric cancer, gastrointestinal cancer, gall bladder cancer, bile duct cancer, colon cancer, rectal cancer, liver cancer, pancreatic cancer, head and neck cancer, esophageal cancer, mesotheolioma cancer, lung cancer including non-small-cell lung cancer, ovarian cancer, endometrial cancer, cervicial cancer, peripheral T-cell lymphoma (PTCL), melanoma, brain tumors, adenocarcinoma, esophageal cancer, and osteosarcoma.

### Brief description of the drawings

Fig. 1 shows the main metabolic pathways by which folate impact on DNA synthesis, repair and methylation.
Fig. 2 shows the development of mean tumor volume over time in the animal groups of example 1, receiving methylene-THF (Modufolin®) (1.7 mg/kg or 45 mg/kg) and vehicle respectively.

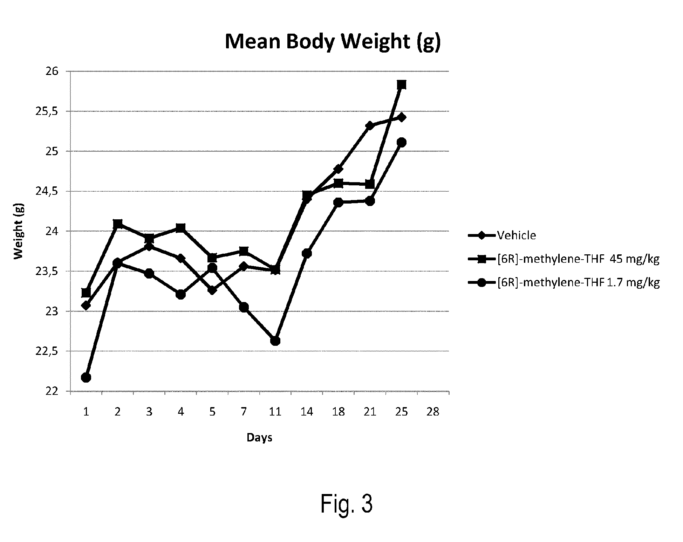

Fig. 3 shows the development of mean body weight over time in the animal groups of example 1, receiving methylene-THF (Modufolin®) (1.7 mg/kg or 45 mg/kg) and vehicle respectively.

### Detailed description

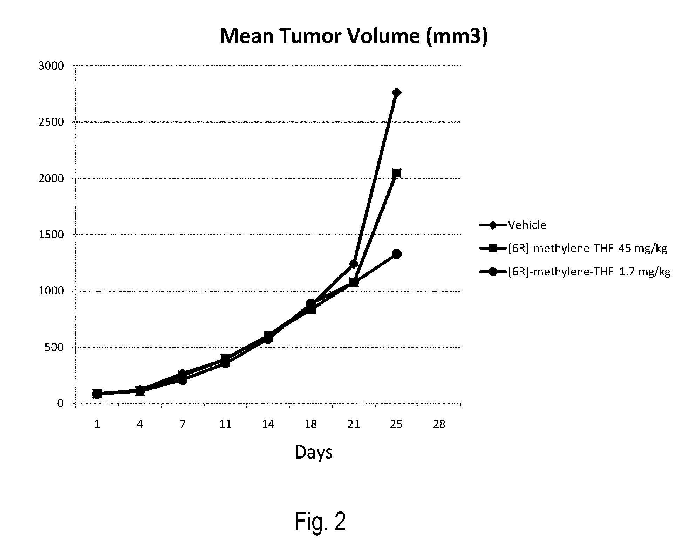

In the research work leading to the present invention, the inventors surprisingly found that methylenetetrahydrofolate (methylene-THF) has an intrinsic tumor inhibiting activity when used as single agent (see example 1, Fig. 2).

As has been described above, folates, such as in the form of leucovorin (folinic acid), tetrahydrofolate (THF) and methylene-THF, are endogenous substances that are essential for cell division and cell growth. For cancer therapy they have been used to alleviate severe side effects of methotraxate and multi-targeting antifolate treatment ((Borsi et al, Pediatric Hematology and Oncology 1990, vol. 7, pages 347-363; EP 1 699 462 B1), and/or to enhance the anti-tumoral effect of 5-FU and analogous substance such as cepecitabine.

Dietary folates are generally available in two supplemental forms, folic acid and leucovorin. Oral administration of folic acid requires a deconjugation step in the small intestine as well as a reduction step into reduced folates in the liver, in order to be available for uptake into cells of the body. Oral administration of leucovorin (also denoted folinic acid or 5-formyltetrahydrofolate) on the other hand, bypasses these deconjugation and reduction steps. Leucovorin seems to be a more metabolically active form of folate than folic acid and, as a reduced folate, might directly cross the blood-brain barrier. Like folic acid, leucovorin must be methylated to become metabolically active. The methylation step of folic acid requires adequate levels of serine and a functional serine hydroxyl-methyl transferase (SHMT) enzyme. Supplementation with the reduced folates methylene-THF and methyl-tetrahydrofolate (methyl-THF) has the advantage of bypassing this methylation step.

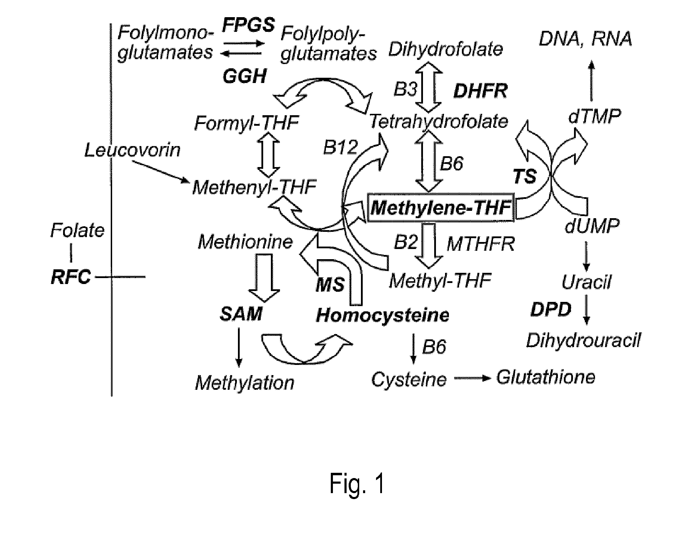

Fig. 1 summarizes the main metabolic pathways by which folate and homocysteine have an impact on DNA synthesis, repair and methylation. The enzyme MTHFR balances the DNA methylation and DNA synthesis pathways to maintain normal homeostasis. (RFC = reduced folate carrier; FPGS = folylpolyglutamate synthase; GGH = γ-glutamyl hydrolase; TS = thymidylate synthase; THF = tetrahydrofolate; MTHFR = methylene-tetrahydrofolate reductase; MS = methionine synthase; SAM = S-adenosylmethionine; DPD = dihydro-pyrimidine dehydrogenase; B2 = riboflavin; B6 = pyridoxine; B12 = cobalamin).

The reduced folate carrier, RFC-1, is the major transporter of reduced folates into the cells (Sirotnak FM, Tolner B, "Carrier-mediated membrane transport of folates in mammalian cells"; Annu Rev Nutr 1999;19:91-122). Intracellularly, reduced folate monoglutamates are converted to polyglutamates by the enzyme folylpolyglutamate synthase (FPGS) (Shane B, "Folylpolyglutamate synthesis and role in the regulation of one-carbon metabolism"; Vitam Horm 1989;45:263-335). The polyglutamated form of tetrahydrofolate is then further converted to 5,10-methylenetetrahydrofolate (methylene-THF), required as a methyl donor in the conversion of dUMP to dTMP (Spears CP et al, "Deoxyuridylate effects on thymidylate synthase-5-fluorodeoxyuridylate- folate ternary complex formation"; Biochem Pharmacol 1989;38:2985-93; Spears CP, et al "Thymidylate synthetase inhibition in malignant tumors and normal liver of patients given intravenous 5-fluorouracil", Cancer Res 1984;44:4144-50). The conversion is catalysed by thymidylate synthase (TS). Methylene-THF is also the precursor of the metabolically active 5-methyltetrahydrofolate (methyl-THF), utilized in the re-methylation of homocysteine. Conversion of methylene-THF to methyl-THF is dependent on the enzyme methylenetetrahydrofolate reductase (MTHFR).

The enzyme y-glutamyl hydrolase (GGH) catalyzes the degradation of inter- and intracellular polyglutamates (Galivan J et al, "Glutamyl hydrolase. Pharmacological role and enzymatic characterization", Pharmacol Ther 2000;85:207-15).

As shown, proper functioning of the DNA synthesis and methylation pathways requires riboflavin (vitamin B2), pyridoxine (vitamin B6), and cobalamin (vitamin B12), in addition to folates. Inadequate levels of any of these metabolites will result in elevated homocysteine levels. Pyridoxine deficiency will also impair the cellular ability to produce glutathione, the master antioxidant needed for detoxification of free radicals and alkylating agent damage.

As is shown in Fig. 1, methylene-THF is an intracellular folate metabolite, for use in thymidylate synthesis by thymidylate synthase (TS). The same is true with respect to the polyglutamates of methylene-THF. Methylene-THF is also used by several other enzymes including methylene-tetrahydrofolate reductase, serine hydroxymethylase and Cl- tetrahydrofolate synthase and methylenetetrahydrofolate dehydrogenase. These interconversions using methylene-THF are essential for purine synthesis, amino acid synthesis, and lipid metabolism. Thus, methylene-THF is located at a metabolic branch point as a substrate for at least four different enzymes (Spears et al; US Patent no 5,376,658).

Thus, methylene-THF has since long been known to propagate cell growth by acting as a one-carbon donor in the conversion between dUMP and dUTP, performed by the enzyme Thymidylate Synthase (TS), and has been expected to have a cancer promoting effect.

As stated above, previous studies have shown that folate status may modulate the toxicity and efficacy of cancer chemotherapy. Thus, folic acid and leucovorin, have been used in combination with anti-cancer drugs, in order to mitigate their cytotoxic effects and/or to increase their anti-cancer effects.

Folic acid, leucovorin and methylene-THF are for instance known to enhance the chemotherapeutic effect of 5-FU. The enhanced anti-cancer efficiency is achieved by methylene-THF, which strengthens the inhibitory effect of 5-FU on thymidylate synthase (TS). Methylene-THF elicits this increased inhibition by forming a ternary complex with TS and FdUMP, which is a metabolite of 5-FU, thereby strengthening the binding of FdUMP to TS. Thus, in the absence of methylene-THF FdUMP binds to TS extremely weakly. However, in the presence of a large excess of methylene-THF even low levels of FdUMP binds to TS. In the presence of excess methyl-THF the ternary complexes between TS, methyl-THF and FdUMP is stable and no significant TS activity occurs. This efficient inhibition of TS leads to a thymidine-less state of the cell, increased uracil misincorporation into DNA and inhibition of tumor cell growth and cell death. Since methylene-THF is the active metabolite of leucovorin metabolism, the use of this endogen folate is much more favorable than the use of folic acid or leucovorin.

Methylene-THF or leukovorin have also been used as rescue agents to methotrexate, in order to alleviate the toxic side effects of methotrexate treatment (see e.g. Borsi et al 1990, Pedriatic Hametology and Oncology, vol. 7, pages 347-363). The mechanism by which the folates antagonize the cytotoxic effects of methotrexate is not known, but the net effect is relief of inhibition of purine and thymidine biosynthesis and a decrease of free methotrexate in the cell. Methylene-THF, when administered with methotrexate, is thus associated with lowered cytotoxicity.

Thus, methylene-THF has previously been shown to have anti-cancer effect in combination with 5-FU by strengthening the binding of FdUMP to TS. Methylene-THF has also been used to mitigate cytotoxic side-effects of antifolates. When administering methylene-THF on its own, i.e. without co-administration of other anti-cancer or chemotherapeutic agents, these mechanisms would not occur and thus it would be expected that methylene-THF would exert its natural function to stimulate cell division and thus growth of cancer cells. The findings of the inventors, that methylene-THF, when used as single agent, inhibits tumor cell growth is therefore very surprising (see example 1).

Thus, for the present invention, methylene-THF and prodrugs or metabolites of methylene-THF are provided for the treatment of cancer. In one embodiment methylene-THF and prodrugs or metabolites of methylene-THF are provided as single agents for the treatment of cancer, i.e. for treatment in isolation of, or separately from, other chemotherapeutic agents.

In connection with the present invention, methylene-THF, tetrahydrofolate (THF) or methyl-tetrahydrofolate (methyl-THF) may be used for the treatment of cancer. Methylene-THF is a folate metabolite that is generally known to exist in equilibrium with THF (see e.g. Fig. 1). Methylene-THF is converted to methyl-THF by the enzyme methylenetetrahydrofolate reductase (MTHFR). Furthermore, methyl-THF is a precursor to THF and methylene-THF.

Alternative expressions for methylene-THF are e.g. 5,10-methylene tetrahydrofolate, 5,10-methylene-THF, CH₂FH₄ and 5,10-CH₂-FH₄. Methylene-THF may e.g. be used in the form of its Ca or Na₂ salt, or in the form of various sulfate salts. Further, according to the present invention, the free acid may be used, i.e. 5,1 0-methylene-tetrahydrofolic acid, or 5,10-methylenetetrahydropteroylglutamic acid (5,10-CHz-H₄-PteGlu).

Alternative expressions for methyl-THF are e.g. 5-methyl-THF, CH₃H₄F and 5-CH₃-H₄F. Methyl-THF may e.g. be used in the form of its Ca or Na₂ salt, or in the form of various sulfate salts. Further, according to the present invention, the free acid may be used, i.e. 5-methyl-tetrahydrofolic acid, or methyltetrahydropteroyl-glutamic acid (5-CH₃-H₄PteGlu).

Alternative expressions for THF are e.g. H₄F, FH₄ and 5,6,7,8-tetrahydrofolate. THF may e.g. be used in the form of its Ca or Na₂ salt, or in the form of various sulfate salts. Further, according to the present invention, the free acid may be used, i.e. tetrahydrofolic acid, or tetrahydropteroyl-glutamic acid (H₄PteGlu).

THF, methylene-THF and methyl-THF may exist in several isomeric forms, however, the biologically active isomers of THF, methylene-THF and methyl-THF, respectively, are preferred according to the present invention. As used herein, "biologically active isomer" relates to an endogen isomer of THF, methylene-THF or methyl-THF, i.e. a natural form which fit into the enzyme systems in the body. One natural, biologically active isomer of THF is [6S]-THF, also denoted [6S]-tetrahydrofolic acid. One natural, biologically active isomer of methylene-THF is [6R]-methylene-THF, also denoted [6R]-5,10-methylenetetrahydrofolic acid (Modufolin^{®}). One natural, biologically active isomer of methyl-THF is [6S]-methyl-THF, also denoted [6S]-5-methyltetrahydrofolic acid.

Methylene-THF comes in different stereoisomeric forms. The racemic mixture comprises [6R, 6S]-methylene-THF (approximately 50% 6R-configuration and 50% 6S-configuration). However, the [6R]-methylene-THF isoform (approximately 100% 6R-configuration) is the biologically active one. For the present invention, [6R]-methylene-THF or [6R, 6S]-methylene-THF may be used. The biologically active isoform, [6R]-methylene-THF, is preferred since it has a more efficient anti-tumoral effect. Natural as well as synthetic methylene-THF or [6R]-methylene-THF may be used. The higher weight portion of a biologically active isomer, the better results will be achieved.

[6R]- methylene-THF for use according to the present invention suitably has a purity of 90% or higher. A purity of 97% or higher is preferred. The higher weight portion of a biologically active isomer, the better stability will be achieved. At low purities, THF, methylene-THF and methyl-THF are very susceptible to oxidation, and thus unstable.

In one embodiment methylene-THF, THF or methyl-THF is administered in isolation from other anti-cancer drugs. In other words, methylene-THF, THF or methyl-THF is in that embodiment not administered simultaneously or concurrently with other anti-cancer drugs. In still another embodiment methylene-THF, THF or methyl-THF is administered to the patient as a single agent treatment. That is, no other pharmaceutically active agent is administered to the patient simultaneously or concurrently with the methylene-THF, THF or methyl-THF.

By being administered as a single agent or in isolation or not being administered simultaneously is meant that methylene-THF, THF or methyl-THF is administered to the patient no later than three weeks, or two weeks, or 7 days, or 5 days or 3 days or 48 hours or 36 hours or 24 hours before the administration of any other anti-cancer drug or any other pharmaceutically active agent. In one embodiment no other anti-cancer drug is administered to the patient before the level in the body of methylene-THF, THF or methyl-THF has sunken below the physiologically active level. The half life of methylene-THF is approximately 20 min, the half life of THF is approximately 30 min and the half life of methyl-THF is approximately 5 hours.

By being administered as a single agent or in isolation or not being administered simultaneously is also meant that methylene-THF, THF or methyl-THF is administered to the patient at the earliest 3 days, or 4 days, or 5 days, or 7 days, or 12 days, or two weeks or three weeks after the administration of any other anti-cancer drug or any other pharmaceutically active agent. In one embodiment the methylene-THF, THF or methyl-THF is administered to the patient after the level in the body of any other anti-cancer drug is below a physiologically active level.

Methylene-THF, THF or methyl-THF may be administered to the patient, e.g. in isolation from other anti-cancer drugs, during a time period of at least one day or at least one week or two weeks or three weeks or one month or two months or three months or six months or one year or two years. In one embodiment it is administered during a time period of at least three months. During said time period, methylene-THF, THF or methyl-THF may be administered to the patient once or twice daily, every second or third day, once to twice a week, or once every second or third week.

Methylene-THF, THF or methyl-THF is preferably administered in a dose of 100 pg to 1000 mg, preferably a dose of 100-200 mg. The dose per day ranges between 1 mg and 1000 mg, particularly between 100 mg and 500 mg. The dose is at least 10 mg/m² (body surface area), preferably at least 20 mg/m², more preferably at least 50 mg/m², most preferably at least 100 or 200 mg/m². The maximum dose is approximately 5 g/m² or preferably 1 mg/m² or 500 mg/m². The dose will be adjusted individually, and may thus vary, e.g. depending on the condition and physiology of the patient.

The dose may be administered e.g. daily, weekly, or monthly. It may, for example, be administered subcutaneously, intramuscularly, intravenously, intraarterial, intraperitoneally, intranasally or orally.

The pharmaceutical compositions according to the invention may also comprise non-active ingredients, such as an inert vehicle, or pharmaceutical acceptable adjuvants, carriers, preservatives, ascorbic acid, reducing agents, ascorbate, antioxidants, etc, which are well known to persons skilled in the art.

The pharmaceutical compositions according to the invention may be formulated by conventional manufacturing methods, such as e.g. by manufacturing methods similar to those used for the production of leucovorin. The methylene-THF of the present invention may for instance be manufactured as described in PCT/EP2004/006944 and patent applications relating thereto.

Examples of cancers to be treated according to the invention are breast cancer, gastric cancer, gastrointestinal cancer, gall bladder cancer, bile duct cancer, colon cancer, rectal cancer, liver cancer, pancreatic cancer, head and neck cancer, esophageal cancer, mesotheolioma cancer, lung cancer including non-small-cell lung cancer, ovarian cancer, endometrial cancer, cervicial cancer, peripheral T-cell lymphoma (PTCL), melanoma, brain tumors, adenocarcinoma, esophageal cancer, and osteosarcoma. However, any cancer may be treated with the pharmaceutical composition according to the present invention.

In one aspect the present invention provides a method for the treatment of cancer comprising administering to a patient a pharmaceutically active amount of methylene-THF, THF or methyl-THF.

It should be understood that the embodiments disclosed in relation to the product aspect of the present invention are, where applicable, relevant also to the method aspect of the invention and vice versa. All characteristics of these compounds that have been described above with respect to the product aspect of the invention thus also apply for the method aspect of the invention and vice versa.

Thus, a preferred embodiment of the method for treatment of cancer comprises administering to the patient the biologically active isomers of methylene-THF, THF or methyl-THF. Most preferably [6R]-5,10 methylene tetrahydrofolate is administered.

In one embodiment of the method the methylene-THF, THF or methyl-THF is administered in isolation from other anti-cancer drugs. In other words, methylene-THF, THF or methyl-THF is not administered simultaneously or concurrently with other anti-cancer drugs. In still another embodiment methylene-THF, THF or methyl-THF is administered to the patient as a single agent treatment. That is, no other pharmaceutically active ingredient is administered to the patient simultaneously or concurrently with the methylene-THF, THF or methyl-THF.

In one embodiment the method comprises administering methylene-THF, THF or methyl-THF to the patient no later than three weeks, or two weeks, or 7 days, or 5 days, or 3 days, or 48 hour, or 36 hours or 24 hours before the administration of any other anti-cancer drug or any other pharmaceutically active agent. In one embodiment no other anti-cancer drug is administered to the patient before the level in the body of methylene-THF, THF or methyl-THF has sunken below the physiologically active level. The method may also or alternatively comprise administering methylene-THF, THF or methyl-THF to the patient at the earliest 3 days, or 4 days, or 5 days, or 7 days, or 12 days, or two weeks, or three weeks after the administration of any other anti-cancer drug or any other pharmaceutically active agent. In one embodiment the methylene-THF, THF or methyl-THF is administered to the patient after the level in the body of any other anti-cancer drug is below a physiologically active level.

In a further embodiment the method comprises administering methylene-THF, THF or methyl-THF to the patient in isolation from other anti-cancer drugs during a time period of at least one day or at least one week to two weeks or three weeks or one month or two months or three months or six months or one year or two years. In one embodiment it is administered during a time period of at least three months. During said time period, methylene-THF, THF or methyl-THF may be administered to the patient once or twice daily, every second or third day or once to twice a week.

In another embodiment the method comprises administering methylene-THF, THF or methyl-THF at a dose that is at least 10 mg/m² (body surface area), preferably at least 20 mg/m², more preferably at least 50 mg/m², most preferably at least 100 or 200 mg/m². The maximum dose is approximately 5 g/m² or preferably 1 mg/m² or 500 mg/m².

The cancer to be treated may be selected from breast cancer, gastric cancer, gastrointestinal cancer, gall bladder cancer, bile duct cancer, colon cancer, rectal cancer, liver cancer, pancreatic cancer, head and neck cancer, esophageal cancer, mesotheolioma cancer, lung cancer including non-small-cell lung cancer, ovarian cancer, endometrial cancer, cervicial cancer, peripheral T-cell lymphoma (PTCL), melanoma, brain tumors, adenocarcinoma, esophageal cancer, and osteosarcoma. However, any cancer may be treated with the pharmaceutical composition according to the present invention

The term "chemotherapeutic agent"or "anti-cancer agent" or "anti-cancer drug" as used herein relates to a medicament for the treatment of cancer, i.e. it relates to an agent/active ingredient having an anti-cancer or anti-tumoral effect. Such an effect may involve inhibition of tumor growth, reduction in tumor volume, induction of tumor cell death, inhibition of formation of metastasis, inhibition of tumor recurrence.

The term "patient" as used herein relates to any human or non-human mammal in need of being treated with the methods or pharmaceutical compositions according to the invention.

The term "treatment" as used herein relates to both treatment in order to cure or alleviate the symptoms of different types of cancer, and to treatment in order to prevent the development of cancer or prevent the recurrence of cancer. In particular, solid tumors are well suited to be treated according to the invention. However, both solid and non-solid tumors may be treated.

The term "pharmaceutically active amount" as used herein relates to a dose of a substance that will lead to the desired pharmacological and/or therapeutic effect. The desired pharmacological and/or therapeutic effect is, as stated above, to cure or alleviate the symptoms of different types of cancer, and to prevent the development of cancer.

The term "physiologically active level" as used herein relates to a level of a substance within the body of a patient, e.g. the circulating level in blood, at which the desired physiological effect is achieved. Below this level the physiological effect is not achieved or is not detectable. The physiological effect of an anti-cancer drug may for instance be inhibition of tumor growth, reduction in tumor volume, induction of tumor cell death, inhibition of formation of metastasis, inhibition of tumor recurrence.

The term "efficacy" of a chemotherapeutic or anti-cancer treatment relates to its ability to affect tumor and cancer cells and to improve the clinical results of the treatment. Such efficacy can be determined e.g. by measuring remission, time to progression, response rate and survival. Methods for measuring such parameters are well-established in the field. For example, anti-tumor effects can be determined by measuring tumor size and tumor size over time.

### Example

**Purpose**: To determine the anti-tumoral efficacy of [6R]-methylene-THF in the HCT116 colorectal carcinoma xenograft model. The HCT116 colorectal carcinoma cell line is sensitive to 5-FU and its analog capecitabine.

**Study design**: A total of 30 female NCr nu/nu mice (10 per group) at the age of 8-12 weeks were injected with 5x10⁶ HCT116 tumor cells in 0% Matrigel sc in flank. When the tumors reached an average size of 80-120 mm² they were administered with intraperitoneal injections of vehicle or [6R]-methylene-THF (Modufolin®), according to the schedule in table 1. Animals were monitored individually. The endpoint of the experiment was when a tumor weight of 1300 mm³ or 65 days was reached, whichever came first. Responders could be followed longer. When the endpoint was reached, the animals were euthanized. Body weight (bw) (g) and tumor volume (mm³) was measured on days 1, 2 (bw only), 3 (bw only), 4, 5 (bw only), 7, 11, 14, 18, 21, 25, 28, 32, 35, 39, 42, 49, 53, 56, 60 and 63.

**Table1. Treatment groups and dosing**

| Group | Vehicle | [6R]-methylene-THF (45 mg/kg, qd x 21) | [6R]-methylene-THF (1.7 mg/kg, qd x 21) |
|---|---|---|---|
| 1 | X | | |
| 2 | | X | |
| 3 | | | X |

### Results

### Tumor volume

The change in mean tumor volume over time is described in figure 1. In the figure it can be seen that compared to the control the [6R]-methylene-THF administrations have a marked influence on tumor volume.

### Body weight

Body weight gives an indication on how severe the side effects of the treatment are, poor development of body weight indicating more adverse side effects. Treatment with [6R]-methylene-THF appears to give a slightly positive body weight development compared to the vehicle group. Low-dose [6R]-methylene-THF appears to have a positive effect on animal weight, while high-dose [6R]-methylene-THF is in line with the vehicle group.

### Conclusion

Very surprisingly, [6R]-methylene-THF has been shown to have an intrinsic antitumor activity in the HCT116 model, both at high and low dose levels. The mechanism of action of this anti-tumor activity is not yet fully understood. Animal body weight measurements also showed that [6R]-methylene-THF was tolerable.

## Claims

1. Methylene-tetrahydrofolate, tetrahydrofolate or methyl-tetrahydrofolate for use in the treatment of cancer.

2. Methylene-tetrahydrofolate, tetrahydrofolate or methyl-tetrahydrofolate for use according claim 1, wherein said methylene-tetrahydrofolate, tetrahydrofolate or methyl-tetrahydrofolate is for administration as a single agent treatment.

3. Methylene-tetrahydrofolate, tetrahydrofolate or methyl-tetrahydrofolate for use according to any one of the claims 1-2, wherein said methylenetetrahydrofolate, tetrahydrofolate or methyl-tetrahydrofolate is for administration in isolation from other anti-cancer drugs.

4. Methylene-tetrahydrofolate, tetrahydrofolate or methyl-tetrahydrofolate for use according to any one of the claims 1-3, wherein said treatment of cancer is inhibition of tumor growth.

5. Methylene-tetrahydrofolate, tetrahydrofolate or methyl-tetrahydrofolate for use according to any one of the claims 1-3, wherein said treatment of cancer is reduction of the volume of a tumor.

6. Methylene-tetrahydrofolate, tetrahydrofolate or methyl-tetrahydrofolate for use according to any one of the preceding claims, wherein said tumor is a solid tumor.

7. Methylene-tetrahydrofolate, tetrahydrofolate or methyl-tetrahydrofolate for use according to any one of the claims 1-6, wherein said methylene-tetrahydrofolate, tetrahydrofolate or methyl-tetrahydrofolate is for administration no later than 24 hours before the administration of any other anti-cancer agent.

8. Methylene-tetrahydrofolate, tetrahydrofolate or methyl-tetrahydrofolate for use according to any one of the claims 1-6, wherein said methylene-tetrahydrofolate, tetrahydrofolate or methyl-tetrahydrofolate is for administration no later than 36 hours before the administration of any other anti-cancer agent.

9. Methylene-tetrahydrofolate, tetrahydrofolate or methyl-tetrahydrofolate for use according to any one of the claims 1-6, wherein said methylene-tetrahydrofolate, tetrahydrofolate or methyl-tetrahydrofolate is for administration no later than 48 hours before the administration of any other anti-cancer agent.

10. Methylene-tetrahydrofolate, tetrahydrofolate or methyl-tetrahydrofolate for use according to any one of the claims 1-9, wherein said methylene-tetrahydrofolate, tetrahydrofolate or methyl-tetrahydrofolate is for administration at the earliest 6 hours after the administration of any other anti-cancer agent.

11. Methylene-tetrahydrofolate, tetrahydrofolate or methyl-tetrahydrofolate for use according to any one of the claims 1-9, wherein said methylene-tetrahydrofolate, tetrahydrofolate or methyl-tetrahydrofolate is for administration at the earliest 24 hours after the administration of any other anti-cancer agent.

12. Methylene-tetrahydrofolate, tetrahydrofolate or methyl-tetrahydrofolate for use according to any one of the claims 1-9, wherein said methylene-tetrahydrofolate, tetrahydrofolate or methyl-tetrahydrofolate is for administration at the earliest 48 hours after the administration of any other anti-cancer agent.

13. Methylene-tetrahydrofolate, tetrahydrofolate or methyl-tetrahydrofolate for use according to any one of the claims 1-9, wherein said methylene-tetrahydrofolate, tetrahydrofolate or methyl-tetrahydrofolate is for administration at the earliest 4 days after the administration of any other anti-cancer agent.

14. Methylene-tetrahydrofolate, tetrahydrofolate or methyl-tetrahydrofolate for use according to any one of the claims 1-9, wherein said methylene-tetrahydrofolate, tetrahydrofolate or methyl-tetrahydrofolate is for administration at the earliest 12 days after the administration of any other anti-cancer agent.

15. Methylene-tetrahydrofolate, tetrahydrofolate or methyl-tetrahydrofolate for use according to any one of the claims 1-14, wherein said methylene-tetrahydrofolate, tetrahydrofolate or methyl-tetrahydrofolate is for administration in isolation from other anti-cancer drugs during a time period of at least 3 months.

16. Methylene-tetrahydrofolate, tetrahydrofolate or methyl-tetrahydrofolate for use according to any one of the claims 1-15, wherein said methylene-tetrahydrofolate, tetrahydrofolate or methyl-tetrahydrofolate is for administration in an amount of at least 10 mg/m², preferably at least 20 mg/m², more preferably at least 50 mg/m², most preferably at least 100 mg/m², 200 mg/m² or 500 mg/m², and at maximum 5 g/m².

17. Methylene-tetrahydrofolate, tetrahydrofolate or methyl-tetrahydrofolate for use according to any one of the claims 1-16, wherein said methylene-tetrahydrofolate, tetrahydrofolate or methyl-tetrahydrofolate is for administration once or twice daily, every second or third day, once to twice a week, or once every second or third week.

18. Methylene-tetrahydrofolate, tetrahydrofolate or methyl-tetrahydrofolate for use according to any one of the claims 1-17, wherein said methylene-tetrahydrofolate, tetrahydrofolate or methyl-tetrahydrofolate is for administration intravenously, orally, intraperitoneally, subcutaneously, intramuscularly, intra-arterially or intranasally.

19. Methylene-tetrahydrofolate, tetrahydrofolate or methyl-tetrahydrofolate for use according to any one of the preceding claims, wherein said cancer is selected from breast cancer, gastric cancer, gastrointestinal cancer, gall bladder cancer, bile duct cancer, colon cancer, rectal cancer, liver cancer, pancreatic cancer, head and neck cancer, esophageal cancer, mesotheolioma cancer, lung cancer including non-small-cell lung cancer, ovarian cancer, endometrial cancer, cervicial cancer, peripheral T-cell lymphoma (PTCL), melanoma, brain tumors, adenocarcinoma, esophageal cancer, and osteosarcoma.
